# EUROPEAN PATENT APPLICATION

(11) **EP 3 683 231 A1**
(43) Date of publication of application: **22.07.2020**
(21) Application number: 19151756.4
(22) Date of filing: 15.01.2019
(51) Int. Cl.: C07K 16/06, B01D 15/18, B01D 15/36, B01D 15/38, C07K 1/16, C07K 1/18, C07K 1/36

(54) **METHOD FOR TRANSFERRING A BATCH PRODUCTION PROCESS TO A CONTINUOUS PRODUCTION PROCESS**

(71) Applicant: Bayer Aktiengesellschaft, 51373 Leverkusen (DE)
(72) Inventor: Schwan, Peter, 51368 Leverkusen (DE); Budde, Bastian, 42096 Wuppertal (DE); Borchert, Sven-Oliver, 51368 Leverkusen (DE); Maiser, Benjamin, 51368 Leverkusen (DE); Classen, Sven, 42096 Wuppertal (DE); Lenz, Jürgen, 42096 Wuppertal (DE); David, Laura, 51061 Köln (DE); Lobedann, Martin, 51061 Köln (DE)
(74) Representative: BIP Patents

(57) **Abstract**

Described herein is a method for transferring of batch production process for a monoclonal antibody to a continuous production process for the same monoclonal antibody.

## Description

Continuous processing for the production of therapeutic proteins gains more and more importance and first solutions for realization of truly continuous, fully automated systems based on disposable equipment have been developed.

However, so far on industrial scale antibodies are manufactured in batch processes. Hence the production protocols and regulatory details only exist for batch production processes.

It was therefore an object of the present invention to provide a method for a reliable and efficient transfer of a batch production process of a given monoclonal antibody to a continuous production process for the same monoclonal antibody.

The invention achieves this object by provision of a method for transferring a batch production process for a monoclonal antibody to a continuous production process for the same monoclonal antibody comprising the steps
a) providing a particle-free fluid (product stream) from a heterogeneous cell culture-fluid mixture containing the monoclonal antibody, in form of a product stream
b) at least one continuous Protein A chromatography, characterized in that aseptic processing is ensured in the continuous mode via sanitization of the Protein A resin with a caustic substance,
c) at least one anion exchange chromatography (AEX) in flow through mode, characterized in that if the flow of the product stream in the batch production process is 1-20 membrane volumes per minute, the flow of the product stream in the continuous production process for a monoclonal antibody is less than 0,1 - 0,99 membrane volumes per minute.

This method has the advantage that it allows for a reliable and efficient transfer of a batch production process of a given monoclonal antibody to a continuous production process for the same monoclonal antibody.

Surprisingly, it was found that all tested product quality attributes und process performance attributes were comparable between batch and continuous processing without any further necessary amendments to the production process.

The described method is not limited to monoclonal antibodies but can also be applied to antibodies in general and possibly to further proteins of interest.

As used herein, the expression "at least one" means one or more.

It is also to be understood that, as used herein the terms "the," "a," or "an," mean "at least one," are understood to encompass the plural as well as the singular and should not be limited to "only one" unless explicitly indicated to the contrary.

As used herein the term "protein" is used interchangeably with the term "protein of interest" and refers to a polypeptide of amino acids. The term encompasses proteins that may be full-length, wild-type, or fragments thereof. The protein may be human, non-human, and an artificial or chemical mimetic of a corresponding naturally occurring amino acid, as well as of naturally occurring amino acid polymers and non-naturally occurring amino acid polymers. The term also encompasses peptides i.e. polymers of amino acids of relatively short length (e.g. less than 50 amino acids). The polymer may be linear or branched, it may comprise modified amino acids, and it may be interrupted by non-amino acids. The term also encompasses an amino acid polymer that has been modified; for example, by disulfide bond formation, glycosylation, lipidation, acetylation, phosphorylation, or any other manipulation, such as conjugation with a labeling component, such as but not limited to, fluorescent markers, particles, biotin, beads, proteins, radioactive labels, chemiluminescent tags, bioluminescent labels, and the like.

Preferably the protein is a therapeutic protein.

As used herein the term "therapeutic protein" refers to a protein that can be administered to an organism to elicit a biological or medical response of a tissue, an organ or a system of said organism.

Even more preferably the protein is an antibody.

The term "antibody" as used herein refers to a binding molecule such as an immunoglobulin or immunologically active portion of an immunoglobulin, i.e., a molecule that contains an antigen-binding site.

Most preferably the protein is a monoclonal antibody.

As used herein, the term "monoclonal antibody" refers to antibody molecules having a single molecular composition, obtained from a population of essentially identical antibodies. This monoclonal antibody shows a single binding specificity and affinity for a specific epitope.

As used herein the term "antibody-drug-conjugate (ADC)" refers to a complex comprising at least one antibody, at least one drug and at least one 'linker' which connects the antibody with the drug.

As used herein the term "antigen-binding antibody fragment" or "antigen-binding fragment" refers to a fragment of an antibody/immunoglobulin (e.g. the variable domains of an IgG) which still comprise the antigen binding domains of the antibody/immunoglobulin. The "antigen binding domain" of an antibody typically comprises one or more hypervariable regions of an antibody.

The terms "biologically active substance", "active substance", "active agent", "drug", or "therapeutic," as used herein refer to any atom and/or molecule, molecular complex or substance administered to an organism for diagnostic or therapeutic purposes, including the treatment of a disease or infection, medical imaging, monitoring, contraceptive, cosmetic, nutraceutical, pharmaceutical and prophylactic applications.

The term "drug" includes any such atom and/or molecule, molecular complex or substance that is chemically modified and/or operatively attached to a biologic or biocompatible structure. The term "prodrug" refers to a drug, drug precursor or modified drug that is not fully active or available until converted in vivo to its therapeutically active or available form.

The term "toxophore" as used herein refers to a chemical group that produces a toxic effect when administered to a cell.

In other words, the active substance can for example be an atom such as a radioactice agent - e.g. a thorium isotope - or an antimitotic agent.

Thus a person skilled in the art immediately recognizes that the methods and devices described herein for an antibody-drug-conjugate are also applicable to a targeted thorium conjugate (TTC).

As used herein the term "small molecule drug" refers to a low molecular weight (<900 daltons) compound that may help regulate a biological process.

As used herein, the term "nucleic acid" refers to deoxyribonucleotides or ribonucleotides and polymers thereof in either single- or double-stranded form. Unless specifically limited, the terms encompass nucleic acids containing analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g. degenerate codon substitutions) and complementary sequences as well as the sequence explicitly indicated.

As used herein the term "particle-free fluid" refers to a product stream, i.e. a cell-free fluid from a heterogeneous cell culture fluid mixture that contains the monoclonal antibody of interest and from which particles of more than 0,2 µm have been removed. In a preferred embodiment said removal of particles is achieved via depth filtration in combination with a 0,2 µm filtration. Alternatively, or in addition other commonly used clarification methods, such as centrifugation, filtration, ATF, flocculation can be used to remove the particles

As used herein the term "batch" refers to a technique, i.e. a mode, of manufacturing proteins such as monoclonal antibodies, in which the protein in question is produced stage by stage over a series of unit operations. All of the material that is to be processed passes a given unit operation before any of said material is processed in the subsequent unit operation.

In some batch production processes the product of a given unit operation, i.e. an intermediate of the production process as a whole, is stored until all of the batch material has been processed by that unit operation. Moreover, the process intermediates can also be stored, e.g. frozen, after the complete material of the given batch has passed the unit operation until the subsequent unit operation is started. The period of time until the subsequent unit operation starts is not always the same.

As used herein the term "continuous" refers to a method for carrying out at least two method steps and/or unit operations in series in which the outlet fluid stream (fluid flow) of an upstream step is transported to a downstream step. The downstream step begins processing the fluid flow before the upstream step is completed. Accordingly, continuous transport or transfer of a fluid flow from an upstream unit to a downstream unit means that the downstream unit is already in operation before the upstream is shut down, i.e. that two units connected in series simultaneously process the fluid flow that is flowing through them.

As used herein the term "aseptic" is used interchangeable with the terms "pathogen-reduced", "microbe-reduced" and "germ-reduced" and means reduced pathogenic count compared to an non-sanitized state and ideally free or freed from pathogens such as microorganisms and viruses. In other words aseptic refers to a state of reduced pathogenic count, i.e. a pathogenic count per area or volume unit of close to zero that is achievable by means of a suitable germ-reducing method, wherein this germ-reducing method can be selected from gamma irradiation, beta irradiation, autoclaving, Ethylene Oxide (ETO) treatment, Ozone treatment, "Steam-In-Place" (SIP) and/or Heat in Place treatment or treatment with sanitization agent like 1 M NaOH.

As used herein the term "fluid stream" or "fluid flow" refers to a flow of liquid and/or gas, which can contain dissolved or partly dissolved species like the monoclonal antibody of interest or its precipitates, salts, sugars and cell components and/ or salts, flocculations, precipitations and/or crystals.

As used herein the term "product stream" is used interchangeably with the term "product flow" and refers to a cell-free fluid from a heterogeneous cell culture fluid mixture that contains the monoclonal antibody of interest. For sake of clarity the product stream is also a "fluid stream" or "fluid flow" in the sense of this description.

As used herein the term "chromatography" refers to the separation of a mixture of two or more analytes into individual components based on the differential distribution of the components between a stationary phase and a mobile phase. The stationary phase can be a resin and/or a membrane absorber.

As used herein the term "flow-through" refers to an operation mode of a chromatographic unit, in which many of the impurities either specifically bind to the separation medium while the product of interest does not, thus allowing the recovery of the desired product in the "flow-through" and/or in which both the product of interest and one or more impurities bind to the separation medium. In the second case the impurities are present to a higher extent in the separation medium than the product of interest and hence as loading continues unbound product of interest can be recovered in the "flow through". In other words, the fluid stream leaving the chromatographic unit operation during the entire time when product is loaded on the chromatographic unit operation constitutes the product stream.

As used herein the term "bind and elute" (or bind-and-elute) refers to an operation mode of a chromatographic unit, in which the product differentially binds to the chromatographic medium. Hence, a bind and elute type chromatography comprises at least the steps of loading, washing, elution and regeneration of a chromatography column, wherein the main constituent of the fluid stream leaving the chromatography column during elution is the product stream.

One type of bind and elute chromatography is continuous bind and elute chromatography where a subsequent unit operation starts processing the product stream before the continuous bind and elute chromatography has finished processing the entire product stream i.e. two units connected in series simultaneously process the fluid flow that is flowing through them.

Surprisingly, in the at least one anion exchange chromatography (AEX) the lower flow of the product stream in the continuous production process for a monoclonal antibody results in less precipitate formation and more host cell protein (HCP) depletion.

Without wishing to be bound by theory the minimization of precipitate formation and increase in HCP depletion can result from the longer contact times of the product stream with the anion exchange chromatography in the continuous process compared with the batch process which in turn leads to a longer precipitation time and a larger precipitation surface.

Thus, a person skilled in the art would also consider lowering the flow of the product stream in the batch process in order to minimize precipitate formation and increase HCP depletion.

As used herein the term load density refers to mass of protein of interest that is loaded onto a given device e.g. onto a chromatography column, a membrane absorber or a filter.

In one embodiment of the method described herein the anion exchange chromatography is characterized in that if the batch production process for a monoclonal antibody comprises a membrane absorber for AEX than in the continuous production process for the same monoclonal antibody said AEX is carried out in a pulsatile manner.

In one embodiment of the method described herein anion exchange is performed employing a number of parallel membrane absorbers. In an alternative embodiment the product stream passes the anion exchange step using a pulsatile manner.

As used herein the term "pulsatile manner" is used interchangeably with the term "staggered manner" and refers to a processing mode in which the product stream of the continuous production process is collected prior to entering a given unit operation in order to enable a higher flow velocity of a given portion of the product stream as soon as enough of the product stream was collected. In other words a given quantity of the product stream is collected and when the predetermined quantity is reached all of said portion of the process stream constituting said quantity enters the subsequent unit operation at once, thereby enabling higher flow velocities in said subsequent unit operation.

Using a pulsatile manner for the anion exchange has the advantage that the already validated membrane filter of the corresponding batch process can be used.

Alternatively, in order to maintain a constant flow velocity in the continuous production process several smaller anion exchange membrane absorbers, which are used in parallel, can be employed, which would need to be regenerated during the continuous production process. However, the above described combination of membrane adsorber used for the batch production process with the temporarily higher flow velocity in the pulsatile manner as already stated has the advantage that membrane absorbers already validated e.g. in terms of viral safety for the batch process can be employed.

In one embodiment of the method described herein at least one filtration providing a filtrate is carried out during the production process.

In a further embodiment the protein A chromatography - via aiming at a constant loading rate - achieves a production rate, which is constant within a range of +/- 50 % in a given time e.g. a cycle time or a complete process time such as 36 h etc.. In other words the average production rate is +/- 50 % e.g. in a cycle time or in a defined amount of time such as an hour or a complete process time etc.

As used herein the "production rate" refers to the average mass of protein of interest, i.e. the monoclonal antibody, present in the product stream in a given amount of time, i.e. gram of protein per hour or gram of protein per cycle time etc.. Also the loading rate is the mass of protein of interest, i.e. monoclonal antibody, present in the product stream in a given amount of time, i.e. gram of protein per hour or gram of protein per cycle time etc.

This embodiment has the advantage that the concentration of the protein of interest, i.e. the monoclonal antibody, in the product stream leaving the continuous Protein A chromatography and thus the product stream of all subsequent unit operations is on average constant within a given time.

In one example 2000 I cell-free harvest from a bioreactor containing the monoclonal antibody of interest, is used and the concentration of the monoclonal antibody of interest in the product stream leaving the continuous Protein A chromatography is 42 g/h. A man skilled in the art is aware, that the concentration per time of a given protein of interest i.e. the monoclonal antibody, depends on said protein of interest i.e. the monoclonal antibody, inter alia its stability characteristics. Thus in another example 120 I cell-free harvest from a bioreactor containing the protein of interest i.e. the monoclonal antibody, is used and the production rate of the protein of interest i.e. the monoclonal antibody, in the product stream leaving the continuous Protein A chromatography is 9.2 g/h.

In one embodiment of the method described herein the, caustic substance used in the continuous protein A chromatography is chosen from the group consisting of 0.01-1.0 M NaOH, 0.01-1 M KOH, 0.5-1 M NaCI, 0.1-1.0 M Na2SO4, 2-6 M Guanidine hydrochloride, 2-8 M Urea, 10-50% isopropanol, 10-50% ethanol, 0.1-5% benzyl alcohol and peracetic acid or combinations thereof.

The use of the caustic substance has the advantage that it ensures yield purity equally well as 25 kGy gamma irradiation in combination with cyclic sanitization with 0.1M NaOH.

In further embodiments of the method described herein, the at least one continuous Protein A chromatography is further characterized in that the flow in continuous mode is 0-8 preferably 0.2 - 3.8 times less than in batch mode and/or wherein the cycles per column in continuous mode is 10-20 times higher than in batch mode.

One way of determining the flow of the protein A chromatography is dividing the volumetric flow rate by the cross section of the chromatography column, i.e. the unit of the flow is m/s or cm/h. For a skilled person it is clear that the contact time of a chromatography can be derived from a given flow and a given column height.

In further embodiments of the method described herein the protein A chromatography is monitored at the beginning of the continuous protein A chromatography, in the middle of the continuous protein A chromatography and at the end of the continuous protein A chromatography via integral sampling in order to determine whether the product stream passing the protein A chromatography at any one time point is to be kept or to be discarded.

As used herein the term "integral sampling" refers to a continuous sample collection throughout a predetermined period of time, i.e. a duration of time.

In some embodiments the integral sample collection is carried out by providing a container with a neutralizing solution into which the integral sample is sampled. This has the advantage that the sample characteristics are maintained until the analysis is carried out even though the sample collection can take some time.

In addition or alternatively, in some embodiments the integral sample is cooled and/or frozen in order to maintain its characteristics until the analysis is carried out.

In a further additional or alternative embodiment the integral sample collection is carried out together with inline quenching whereby simultaneously with the sample a stabilizing and/or neutralizing agent is added to the sample container.

In a preferred embodiment of the integral sampling the integral sampling is carried out at the beginning of the continuous protein A chromatography, in the middle of the continuous protein A chromatography and at the end of the continuous protein A chromatography.

For example, during start-up integral sampling was carried out for one cycle of the protein chromatography. In another example, an integral sample was taken for the time in which a specific predetermined volume such as 2-2.5 column volumes was eluted from a column in a bind and elute chromatography step.

In a further preferred embodiment the sample collection for the integral sampling of the continuous protein A chromatography is carried out after the portion of the product stream to be sampled has passed the protein A chromatography e.g. right before the product stream enters the subsequent unit operation and the collected sample is immediately neutralized.

This embodiment has the advantage that the sample represents the product stream. In one example the sample is neutralized using citrate buffer.

In contrast to an integral sample a grab sample, is an immediate sample also termed momentary sample herein.

In another preferred embodiment of the method described herein the method comprises at least one cation exchange (CEX) chromatography step in parallel batch mode, wherein the number of chromatography columns used in the CEX step to carry out the parallel batch mode is chosen in such a manner that the time required for regeneration and elution of a given number of columns is smaller than the load time of a given column, thereby ensuring that always at least one column can be used for loading and thus achieving a continuous loading product stream.

The use of parallel batch mode has the advantage that the process conditions of the batch process can be directly applied to the continuous process.

In the context of a chromatography parallel batch, refers to a multicolumn chromatography device enabling a continuous loading without overloading individual columns. In order to enable the product stream to continuously enter said device the parallel batch multicolumn chromatography device has a minimum number of columns enabling a continuous loading without overloading the columns.

Thus, more columns are needed for processing the same amount of monoclonal antibody of interest in parallel batch mode than in batch mode. Moreover, also more columns are needed in parallel batch mode than when using a continuous chromatography such as a BioSMB, since in parallel batch mode no overloading of the column occurs.

In a preferred embodiment the number of chromatography columns used in the CEX step to carry out the parallel batch mode is thus in the range of 2-8, preferably 4.

In a further preferred embodiment, the dimensions of the CEX chromatography are chosen so that the flow of the product stream of the continuous process is within a range of 50% - 200% of the flow of the product stream of the batch process.

This connection of the process conditions of the continuous process to the process conditions of the batch process allows to use the process optimization regimes developed for a given batch process for the production of a specific monoclonal antibody of interest also for the continuous process for the production of the same specific monoclonal antibody of interest.

In one embodiment of the method descried herein, the at least one continuous Protein A chromatography is further characterized in that no peak cutting is performed during elution. In an alternative embodiment peak cutting is performed in continuous Protein A chromatography.

In a further embodiment of the method described herein the peak cutting conditions developed for the CEX step in batch mode are also applied in the continuous mode.

As used herein the term "peak cutting" or "peak cutting conditions" refers to a method in which only a certain fraction of the eluate is captured and the rest is discarded. Via discarding fractions of the eluate which contain less product or product of potentially less quality - such as the very first and very last eluate fractions - overall product quality is improved. In order to determine which fractions to discard for example monitoring of the eluate at a wavelength where the protein of interest absorbs passing light, e.g. 280 nm can be used. For example when the peak of the UV signals starts collection of that fraction of the product stream is started, as this portion of the product stream contains the most of the protein of interest, and when the peak ends collection of the portion of the product stream ends.

Thus, via applying the peak cutting criteria of the batch process to the continuous production process of the same protein of interest, a similar protein quality in the two processes can be achieved.

In a further embodiment of the method described herein aseptic processing of the cation exchange chromatography processing is ensured in the continuous mode via sanitization of the CEX resin with a caustic substance.

In a further embodiment of the method described herein the continuous process is carried out in a closed system using disposable equipment.

As used herein the term "closed" refers to both "functionally closed" as well as "completely closed".

As used herein the term "completely closed" means that the production plant is operated in such a way that the fluid stream is not exposed to the room environment. Materials, components, objects, buffers, and the like can be added from outside, wherein, however, this addition takes place in such a way that exposure of the fluid stream to the room environment is avoided.

The term "functionally closed" refers to a process that may be opened but is "rendered closed" by a cleaning, sanitization and/or sterilization that is appropriate or consistent with the process requirements, whether sterile, aseptic or low bioburden. These systems shall remain closed during production within the system. Examples include process vessels that may be CIP'd and SIP'd between uses. Non-sterile systems such as chromatography or some filtration systems may also be rendered closed in low bioburden operations if appropriate measures are taken during the particular system setup.

In a further aspect what is described herein relates to a method for transferring a batch chromatography process for a protein of interest to a continuous chromatography process for the same protein of interest, wherein at least one continuous bind and elute type chromatography in the continuous process is monitored using asymmetry factor analysis.

In general, asymmetry factor (As) of a chromatography column is used as a parameter for the evaluation of the packing state of a chromatography column. A well packed column yields higher resolution and yield. For the calculation of "As", an evaluation substance is injected onto a packed column, and "As" is calculated from the resulting chromatogram. Detection of the evaluation substance is possible by using an ultraviolet absorption (UV) detector, an electrical conductivity detector, a differential refractive index detector or the like. The resulting peak is analyzed and As calculated with the following equation.

**Aₛ = b/a** Where: a is the peak width (left half) at 10% of peak height, b is the peak width (right half) at 10% of peak height

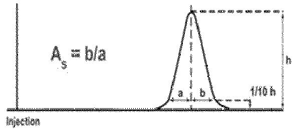

Apart from asymmetry factor determination chromatography column packing efficiency can be checked by calculating Height Equivalent Theoretical Plates (HETP).

In batch chromatography processes the asymmetry factor is usually employed in order to decide whether a given column meets the requirements set for chromatography columns used in a given production process.

In a continuous process, bind-and-elute type chromatography columns are often used for many cycles. Examples for such a scenario are the use of a continuous chromatography for example a BioSMB device or the parallel batch set up described above. Thus, after the first cycle of loading, binding, washing and eluting a chromatography column in a continuous process the asymmetry factor of a given column might have changed.

In order to be able to account for changes of the asymmetry factor during continuous bind-and-elute chromatography the inventors of the current invention have to their knowledge monitored for the first time at least one continuous bind and elute type chromatography using asymmetry factor analysis.

In one embodiment of the asymmetry factor analysis used for monitoring at least one continuous bind and elute type chromatography a detector measures an UV Signal preferably at 280 nm or 300 nm. The signal is measured first when the column is subjected to with a buffer with a high conductivity and again when afterwards the column is subjected to a buffer with low conductivity. In this embodiment these two applications of the respective buffers of high and then low conductivity are carried out prior to elution of the column.

The measured asymmetry factor can be used to relate any product quality findings to the packing state of the column and/or to decide when a given column needs to be replaced in order to maintain product quality. Typically an asymmetry factor of around 1.0 is employed.

In one embodiment of the method described herein it was surprisingly found that a bind-and-elute chromatography column preferably a continuous bind-and-elute chromatography column with an asymmetry factor in the range of 1.5-1.9, preferably of 1.7 had no adverse effect on product quality.

A person skilled in the art knows that the above given asymmetry factor values can differ for each type of chromatography.

In general both during a batch and/or a continuous process the harvest starting material can be stored at 2-8°C and can then equilibrated to room temperature before processing or can be processed directly e.g. via directly starting with a protein A chromatography on the chilled material. Furthermore it is also possible to equilibrate the harvest material, i.e. the process stream following the protein A unit operation.

Moreover, in a variant especially of the continuous process a module for inline gas addition is provided following a debubbling module.

### Brief description of the figures

Figure 1 shows the process overview for the exemplary continuous DSP including sampling spots and techniques

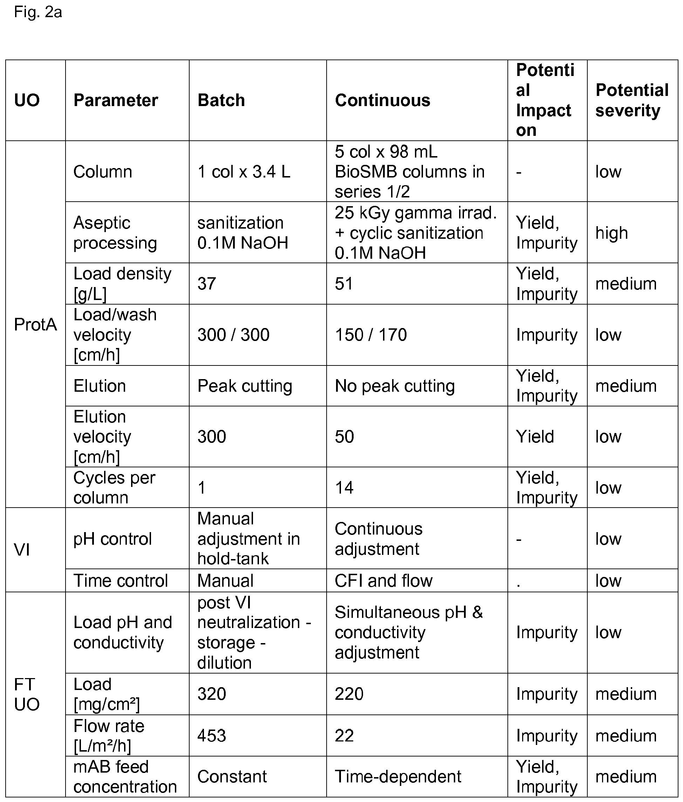

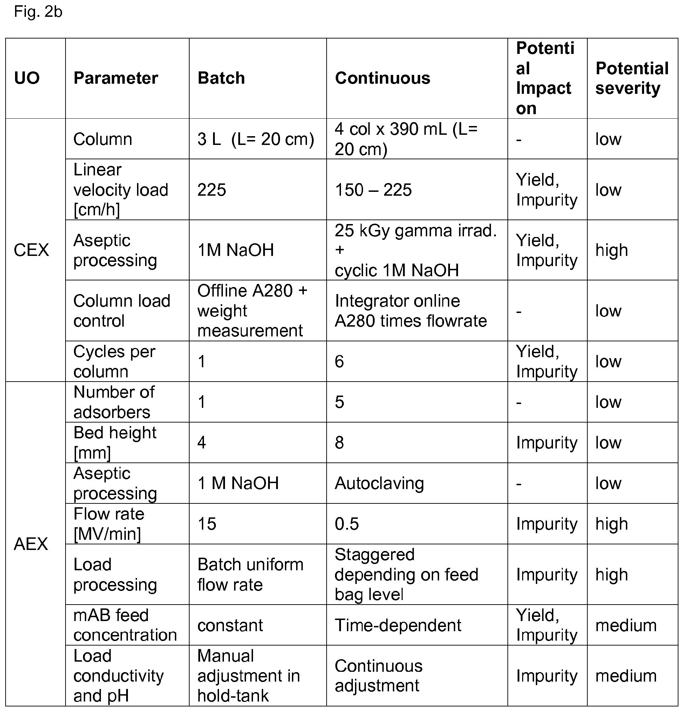

Figures 2a and 2b show for one example process which was transferred from batch to continuous mode the differences in batch and continuous DSP and possible impact on yield, product quality and impurities

### Example

### 1. General

In general after establishing the method of transferring of the batch production process for a monoclonal antibody to a continuous production process for the same monoclonal antibody both process modes i.e. batch and continuous were compared experimentally by purifying the protein of interest, here a monoclonal antibody, from the same harvest material.

For the continuous process thus a combination of a fed-batch USP - as employed for the batch process - and a continuous down-stream process (DSP) were used. This variant of the continuous process mode is known as a so-called hybrid process. However, a method for a reliable and efficient transfer of a batch down-stream production process of a given monoclonal antibody to a continuous down-stream process production process for the monoclonal antibody did not exist so far.

Samples were drawn at different time points and positions in the process for batch and continuous mode. Product quality attributes und process performance attributes were determined. The resulting polished material was processed to drug substance and further evaluated regarding storage stability and degradation behavior. Minor differences between batch and continuous samples were expected as different processing conditions were unavoidable due to the different nature of batch and continuous processing. All tests revealed no significant differences in the intermediates and comparability in the drug substance between the samples of both process modes. The stability study of the final product also showed no differences in the stability profile during storage and forced degradation.

Therefore, the work described herein does not only provide a method for a reliable and efficient transfer of a batch production process of a given monoclonal antibody to a continuous production process for the same monoclonal antibody but also demonstrated that the process mode does not influence product quality which is an essential prerequisite for successful implementation of the technology at every stage of the product life cycle

### 2. Materials

Harvest was generated in a 200 L Sartorius fed-batch reactor (Sartorius Stedim Biotech, Göttingen, Germany) using a Chinese hamster ovary (CHO) cell culture. The fermenter was harvested using a Pall Stax depth filter (Pall GmbH, Dreieich, Germany). The 0.2 µm filtered product had an IgG titer of 2.8 g/L.

GE MabSelect Sure was used as Protein A (ProtA) capture resin (GE Healthcare Life Sciences, Little Chalfont, UK). GE Capto SP impres was used in the intermediate bind & elute (B/E) chromatography process. Sartorius Sartobind Q membrane adsorber capsules were installed as final polishing step.

Citric buffers were employed for all chromatography steps. In case of continuous processing, buffers were 0.2 µm filtered into 200 L Sartorius Flexboy bags. Intermediates for batch processing were 0.2 µm filtered using Sartorius Sartopore 2 filters before storage. Sartorius Sartoguard NF filters were used as intermediate filters in continuous production to remove precipitates.

Pall BioSMB PD systems (Pall Biotech, Dreieich, Germany) were utilized for continuous chromatography, whereas Äkta process systems (GE Healthcare Life Sciences, Little Chalfont, UK) were used for batch chromatography.

### 2.2. Analytics

Important product attributes were determined offline from samples drawn at different positions and time points within the batch and the continuous process. The product concentration was either determined by POROS-A high performance liquid chromatography (HPLC) or, after the ProtA capture step, through the determination of the absorbance at 280 nm wavelength (A280). The concentration of monomer, high molecular weight (HMW) and low molecular weight (LMW) isoforms was determined by size exclusion chromatography (SEC) HPLC. As the resolution for LMWs were not sufficient with this method, only the results for monomer concentration and HMWs were further used. In order to evaluate the degree of fragmentation of the product into light and heavy chain (LC and HC) during the process, capillary gel electrophoresis under non-reducing conditions was performed (CGE-nr). Moreover, CGE under reducing conditions was conducted to determine the degree of fragmentation into smaller debris (CGE-r). The charge variant distribution was investigated by capillary isoelectric focusing (clEF). The impact of the process mode on the protein oxidation was determined by idES-HPLC. The activity of the mAb was investigated through a binding enzyme-linked immunosorbent assay (ELISA). The n-glycan profile was investigated with the help of HILIC uHPLC using Glykoprep®-plus Rapid N-Glycan sample preparation with the InstantAB kit (GPPNG-LB).

Additionally, process-related impurities such as host cell proteins (HCPs), deoxyribonucleic acid (DNA) and leached Protein A were determined. DNA was detected by polymerase chain reaction (PCR), whereas HCPs and leached ProtA were investigated through different ELISA methods.

Finally, the bioburden of the process in batch and continuous mode was determined. Therefore, the total aerobic microbial count (TAMC) and the total yeast/ mold count (TYMC) were measured. Additionally, the endotoxin level was identified according to the harmonized testing method of Ph.Eur., USP, JP and TGA. The test kit N588 Pyrogent-5000 from Lonza was used for quantification

### 2.3. Plant setup

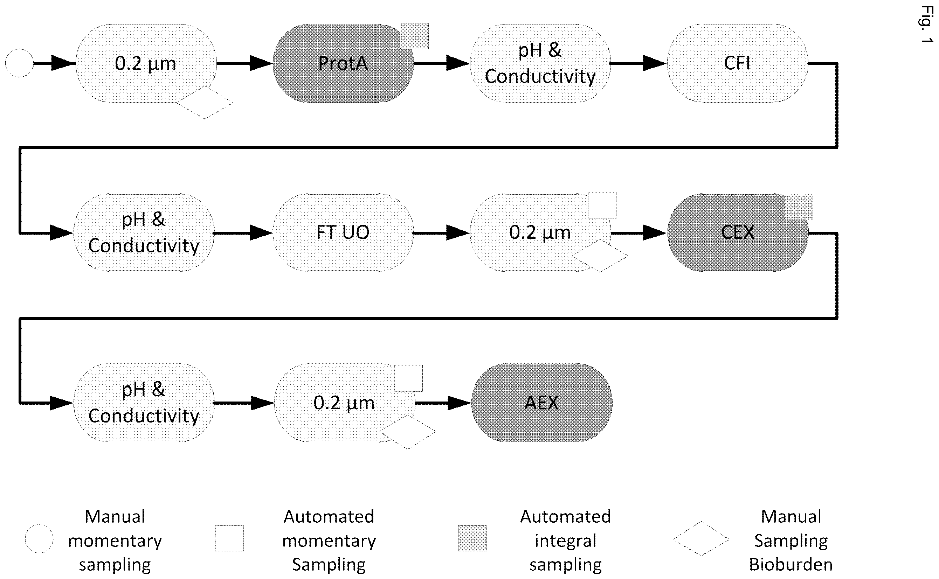

The steps that were transferred from batch to continuous were capture (ProtA), low pH viral inactivation, intermediate chromatography by cation exchange (CEX) and polishing chromatography by anion exchange (AEX). Fig. 1 shows the process overview for the continuous DSP including sampling spots and techniques. Prior to the first chromatography step, the harvest was pre-filtered by a 0.2 µm filter. The ProtA chromatography was executed in B/E mode as in the batch process, but by using a BioSMB chromatography with 5 columns a continuous feed stream and a continuous eluate stream is generated. After the ProtA step, pH was adjusted for the following low pH viral inactivation (VI). In order to generate the necessary hold time for the step, a coiled flow inverter (CFI) was introduced into the system. Afterwards, pH and conductivity were adjusted for the following flow through (FT) UO and intermediate CEX. Another filtration step took place prior to the intermediate chromatography step. The CEX was executed in parallel batch mode to enable peak cutting to gain the required product purity in this step. The CEX eluate was again adjusted regarding pH and conductivity and 0.2 µm filtered prior to the polishing AEX in FT mode. The material after the polishing AEX chromatography of both process modes was further processed through VF and ultrafiltration/diafiltration (UF/DF), all in batch mode.

In the continuous DSP, pH and conductivity were inline-monitored and feedback-controlled if needed. UV was only inline monitored. All modules were highly automated and synchronized using a PCS7 distributed control system. A steady-state fluid connection existed between all modules and the entire process was a closed system. Sampling for bioburden and instantaneous grab samples were executed in either manual or automated mode; sampling for integral samples was achieved in automated mode. Grab samples were taken in a very short time and are only representative for the current status, whereas integral samples were drawn over a long period of time and were thus representative for this prolonged time.

In the batch DSP, pH and conductivity adjustments were performed manually. No fluid connection existed between the different UOs. Samples were taken manually from the pooled process intermediate between the different UOs. As the purification process took several days, process intermediates were stored at 2-8 °C between UOs, whereas no such process hold times exist in the continuous process.

In the batch process, all chromatography steps were sanitized with sodium hydroxide. In the continuous process, the majority of the parts were gamma irradiated with at least 25 kGy, including the chromatography columns. Where no gamma irradiation was applicable, the parts were treated with ethylenoxide (EO) or autoclaved before introduction into the system.

### 3. Experimental procedure

In order to preclude the impact of storage on the starting material, the harvest stored for 5 days at 2-8°C was split into two parts for the two process modes immediately before processing. The harvest part for the batch purification was equilibrated to room temperature and processed on the ProtA column within approximately 2 h. The batch production until the AEX step was carried out in 7 days comprising intermediate storage at 2-8°C. Batch chromatography processes followed standard procedures for installation and processing. This included the sanitization prior to each step with sodium hydroxide.

During the continuous DSP, the first step was the inline calibration and adjustment of the pH and conductivity sensors inline in the conditioning modules using citrate buffers at the operating point. The entire DSP was then primed with process buffers and controlled with respect to flow, pH and conductivity. Once steady state conditions were reached, the harvest was connected to the first filtration module of the DSP. During the whole continuous production time (40 h), the harvest was supplied out of a chilled vessel. The feed material was equilibrated to room temperature during the initial 0.2 µm filtration before entering the ProtA UO.

The shut-down phase was initialized by replacing the harvest bag with ProtA equilibration buffer. ProtA chromatography operation continued until all 5 columns were eluted. After stopping the ProtA module, a low pH buffer flush at the inlet of the VI module continued chasing product. CEX and AEX chromatography continued until the inlet protein concentrations were below 0.1 g/L. The process was fully automated including start-up, shut-down and handling of events. The established parameter control strategy ensured that only product within normal operating ranges (NORs) was further processed.

Process parameters were kept identical between batch and continuous production wherever possible. This includes chromatographic buffers, resins and membrane types. Furthermore, column load conditions with respect to conductivity and pH were the same.

### 4. Process control

As similar sensors were used for batch and continuous processing, the measurement accuracy in both process modes was comparable. For the conductivity sensors, an accuracy of ± 5% was assumed, whereas the accuracy for pH meters in general is ± 0.1 pH units. During the continuous process, sensors were adjusted every 72 h before and after processing, enabling the identification of the sensor drift. For pH sensors, the maximum drift was 0.076. The conductivity sensors drifted by 0.02 mS/cm at maximum

Consequently, the process control accuracy shown in this study guarantees the same process conditions for the entire DSP material based on the collected measurement data

### 5. Quality Attributes

In order to ensure side-by-side comparability between batch and continuous downstream processing, the product quality attributes must be similar through all stages of the process and over the entire production time taking expected differences into account. Therefore, several parameters such as product concentration, product-related impurities and process-related impurities were analyzed for the samples drawn from the batch and the continuous process.

In order to evaluate the comparability between both process modes, the final drug products were extensively tested and compared to each other. Moreover, a stability study was performed with the final drug product to evaluate the comparability of storage and degradation behavior. Finally, the microbial load of both process modes was tested.

Additionally, the microbial load of both process modes was tested. Finally, a stability study was performed with the final product to evaluate the comparability of storage and degradation behavior.

### 6. Process intermediates

Sampling took place before and after every chromatography step in batch as well as in continuous mode.

Comparing batch and continuous results, differences in the mAb concentration were apparent. The highest differences were observed at the AEX FT samples. This process behavior was expected and the explanation lies in the combination of CEX and AEX attributes. Lower protein concentrations in the CEX eluate are observed because of a larger elution volume of column no. 1. Lower concentration in the AEX pool was attributed to a longer chase at the end of an AEX cycle, whereas in batch the chase was fractionated according to an A280 inline signal.

Regarding the monomer ratio, minor differences between the different process steps were observed. This can be explained by the low resolution of this method for monomer and LMWs. Regarding the HMWs there are no significant differences between the batch and continuous processes within the assay variability.

The results of CGE under reducing and non-reducing conditions showed that IgG purity remains constant throughout the purification process and is comparable with the batch data. The differences are within the assay deviation.

Additionally to the results for product-related impurities, the charge heterogeneity was evaluated with the help of clEF. No differences between the different samples could be observed over the entire process of the two process modes. The charge variant distribution was stable.

Concerning the process-related impurities it was found that the initial ProtA concentration was higher for the continuous process than for the batch process. This could be caused by the different sanitization methods used. The batch column was sanitized once with 0.1 M NaOH. The columns used for the continuous process were gamma-irradiated once and sanitized with 0.1 M NaOH in every cycle. Additionally, more cycles were used in the continuous DSP and the collected elution volume was higher than in the batch DSP. In both process modes the leached ProtA concentration decreased rapidly due to the FT UO before the CEX. Afterwards, the ProtA concentration was stable close to the limit of quantification for the used assay.

Also the host cell DNA concentration over the course of the process was measured (data not shown). Comparing the four continuous and the batch samples to each other, the curves were almost identical. The major depletion of DNA took place during the ProtA chromatography step. The remaining DNA was then removed by the FT UO afterwards. Consequently, no DNA was detected in the CEX Load samples and beyond.

Measurement of the HCP concentration over the course of the process showed that the main reduction of HCPs took place during the ProtA chromatography, followed by the FT UO and CEX. The analysis results of ProtA Load and Eluate were almost identical. Beginning with the CEX Load samples, the results of the four continuous and the batch sample differed. As the specific load of mAb and the flow rate on the FT UO was higher in batch mode than in continuous mode the HCP breakthrough was higher as well. Nevertheless, the resulting final measurements of the AEX FT samples showed similar low HCP concentrations.

To conclude, all tested product parameters and process-related impurities showed only minor differences between batch and continuous processing and between continuous processing at different time points. The occurred alterations were explainable by differences in sanitization methods or process parameters. This proved that batch and continuous downstream processing of the mAb results in a comparable product over the entire course of the process.

### 7. Final Drug Substance

In order to evaluate the comparability of the final drug substance, the final product was extensively tested.

The post-AEX process intermediates of both process modes were further processed to bulk drug substance via viral filtration, ultrafiltration and diafiltration. The resulting material was further analyzed for product quality attributes such as charge variants, pl range, n-Glycan profile, activity, molecular weight, idES-HPLC and IgG purity. Additionally, product related impurities were analyzed as well. It was clearly demonstrated that most results are identical or only show minor differences. The same quality of results was achieved for all other tested attributes as well (data not shown). All samples were within the given specifications. Tests for host cell DNA, endotoxins and microbial load were negative.
To conclude, the results of the final drug substance for batch and continuous processing show full comparability between both process modes. Consequently, the study design and execution can be regarded successful.

### 8. Stability studies final drug substance

Stability tests were conducted at room temperature (18-26 °C) and 40°C for 4 weeks each.. The final product was stored in sterile low density polyethylene (LDPE) bags during the study. Several quality attributes were tested during the study, including protein concentration (OD A280), monomer and HMW concentration via SEC-HPLC, %intact IgG through CGE-nr, charge variant distribution (%main peak, % total basic and acidic isoforms) with the help of clEF, the impact on protein oxidation (idES-HPLC) and finally activity through binding-ELISA. Exemplary, the results for the charge variants distribution did not show any significant impact on the charge heterogeneity. Moreover, no difference between batch and continuously produced mAb solution were observed. Also at elevated temperature (40°C) no significant difference between batch and continuous samples was observed. To conclude, the stability study showed that also the degradation profile of the final product was comparable between batch and continuously produced mAb considering charge heterogeneity.

Regarding the other tested quality attributes including protein concentration, monomer concentration, HMWs, protein oxidation and activity, no significant difference between both samples was observed at room temperature as well as at 40°C. Consequently, the comparability of batch and continuously produced final drug substance was also shown in a stability study including a wide range of relevant quality attributes.

### 9. Microbial control

In order to evaluate the bioburden of the processes, the TAMC and TYMC were determined from certain samples (see Figure 1 for bioburden sampling positions). Additionally, the endotoxin level was measured. In the continuous process the samples were derived from the retentate side of the 0.2 µm filters, as possible microbial contaminations would concentrate at these positions. For all tested samples, the results for TAMC, TYMC and endotoxin were below the limits of quantification. This leads to values < 1 cfu/100 mL for TAMC and TYMC and < 1.0 EE/mL for endotoxin. Consequently the batch intermediates as well as the entire continuous process can be considered as bioburden-free during the current runs.

### 10. Conclusion

Overall the above results demonstrate that by employing the method of transfer of batch production process for a monoclonal antibody to a continuous production process for the same monoclonal antibody described herein leads to comparable results regarding product quality attributes. Thus, said method represents a major step in the implementation process towards continuous production of mAbs, as already existing batch processes can be transferred into their continuous mode without affecting the comparability of final drug substance characteristics. Therefore, choosing the process mode individually and flexible independent from the product life cycle, from clinical trials material to large-scale production, should be possible in the future.

## Claims

1. Method for transferring of batch production process for a monoclonal antibody to a continuous production process for the same monoclonal antibody comprising the steps
a) providing a particle-free fluid (product stream) from a heterogeneous cell culture-fluid mixture containing the monoclonal antibody, in the form of a product stream,
b) at least one continuous Protein A chromatography, **characterized in that** the aseptic processing is ensured in the continuous mode via sanitization of the Protein A resin with a caustic substance,
c) at least one anion exchange chromatography (AEX) in flow through mode, **characterized in that** the if the flow of the product stream in the batch production process is 1-20 membrane volumes per minute, the flow of the product stream in the continuous production process for a monoclonal antibody is less than 0,1 - 0,99 membrane volumes per minute.

2. Method according to claim 1, wherein the anion exchange chromatography is **characterized in that** if the batch production process for a monoclonal antibody comprises a membrane absorber for AEX then in the continuous production process for the same monoclonal antibody said AEX is carried out in a pulsatile manner.

3. Method according to claim 1 or 2, wherein at least one filtration providing a filtrate is carried out during the production process.

4. Method according to claim 1, wherein the at least one continuous Protein A chromatography is further **characterized in that** the flow in continuous mode is 0-8, preferably 0.2 - 3.8 times less than in batch mode and/or wherein the cycles per column in continuous mode is 10-20 times higher than in batch mode.

5. Method according to claim 1, further comprising at least one cation exchange (CEX) chromatography step in parallel batch mode, wherein the number of chromatography columns used in the CEX step to carry out the parallel batch mode is chosen in such a manner that the time required for regeneration and elution of a given number of columns is smaller than the load time of a given column, thereby ensuring that always at least one column can be used for loading and thus achieving a continuous loading product stream.

6. Method according to claim 1, wherein the at least one continuous Protein A chromatography is further **characterized in that** no peak cutting is performed during elution.

7. Method according to claim 1, wherein the peak cutting conditions developed for the CEX step in batch mode are also applied in the continuous mode.

8. Method according to anyone of the preceding claims, wherein the continuous process is carried out in a closed system using disposable equipment.

9. Method for transferring a batch chromatography process for a protein of interest to a continuous chromatography process for the same protein of interest, wherein at least one continuous bind and elute type chromatography in the continuous process is monitored using asymmetry factor analysis.
